(19) Europäisches Patentamt
European Patent Office
Office européen des brevets

(11) EP 2 533 768 B1

(12) **EUROPEAN PATENT SPECIFICATION**

(45) Date of publication and mention
of the grant of the patent:
**22.07.2015 Bulletin 2015/30**

(21) Application number: **11742895.3**

(22) Date of filing: **11.02.2011**

(51) Int Cl.:
**A61K 9/20** (2006.01)   **G01N 33/94** (2006.01)

(86) International application number:
**PCT/US2011/024591**

(87) International publication number:
**WO 2011/100588 (18.08.2011 Gazette 2011/33)**

(54) **METHODS OF NORMALISING MEASURED OXYCODONE CONCENTRATIONS AND TESTING FOR NON-COMPLIANCE WITH A TREATMENT REGIMEN**

VERFAHREN ZUR NORMALISIERUNG VON GEMESSENEN OXYCODONKONZENTRATIONEN UND ZUR ÜBERPRÜFUNG AUF NON-COMPLIANCE BEI EINEM BEHANDLUNGSPLAN

MÉTHODES PERMETTANT DE NORMALISER DES CONCENTRATIONS D'OXYCODONE MESURÉES ET DE DÉTECTER LA NON-OBSERVATION D'UN TRAITEMENT THÉRAPEUTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.02.2010 US 303467 P**

(43) Date of publication of application:
**19.12.2012 Bulletin 2012/51**

(60) Divisional application:
**15171980.4**

(73) Proprietor: **Ameritox Limited Partnership Baltimore, MD 21202 (US)**

(72) Inventors:
• **LEIDER, Harry Baltimore, MD 21202 (US)**
• **LINDEN, Ariel Baltimore, MD 21202 (US)**

(74) Representative: **Wakerley, Helen Rachael Reddie & Grose LLP 16 Theobalds Road London WC1X 8PL (GB)**

(56) References cited:
**US-A- 5 908 788        US-A- 5 908 788
US-A1- 2003 170 651    US-A1- 2009 291 468
US-A1- 2009 291 468**

• **M. E.M. LARSON ET AL: "Quantification of a Methadone Metabolite (EDDP) in Urine: Assessment of Compliance", CLINICAL MEDICINE & RESEARCH, vol. 7, no. 4, 1 December 2009 (2009-12-01), pages 134-141, XP055065087, ISSN: 1539-4182, DOI: 10.3121/cmr.2009.859**
• **COUTO JOSEPH E ET AL: "Use of an algorithm applied to urine drug screening to assess adherence to an oxycontin regimen", JOURNAL OF OPIOID MANAGEMENT,, vol. 5, no. 6, 1 November 2009 (2009-11-01), pages 359-364, XP009132311,**

EP 2 533 768 B1

**Description**

FIELD OF THE INVENTION

The present invention relates to methods for reducing a risk of drug misuse in a subject, wherein the drug comprises oxycodone, controlled-release oxycodone or a metabolite of oxycodone by, inter alia, testing a urine from said subject.

BACKGROUND OF THE INVENTION

Although hydrocodone stands as the most prescribed opioid in the United States, the opioid that is responsible for the most emergency department (ED) visits in the United States is oxycodone. According to the Drug Abuse Warning Network, approximately 77,000 ED visits in 2007 were due to the nonmedical use of oxycodone. The 2007 National Survey on Drug Use and Health estimates that 4.3 million Americans will abuse OXYCONTIN® sometime during the course of their lifetime. Given the propensity for abuse of oxycodone containing medications and high incidence of ED visits associated with abuse, monitoring patients for compliance while being prescribed a pain regimen is an important component of their care.

Because of known dependency risks, subjects on opioid therapy regimens are typically screened periodically to monitor compliance and efficacy of the prescribed therapy. Due to the limits of known screening techniques, however, subjects misusing the prescribed opioid often pass basic screening tests performed at a clinic and continue to receive the opioid. Furthermore, patients treated with opioids for the management of chronic pain also have been documented to under- report their use of medications. As a result, health care professionals often use external sources of information such as interviews with the subject's spouse and/or friends, review of the subject's medical records, input from prescription monitoring programs, and testing of biological samples (e.g., fluids) to detect misuse of drugs and non-compliance with the prescribed opioid regimen.

Known drug screening methods generally can detect the presence or absence of a drug in a sample. Samples of fluids are generally obtained from the subject, for example, urine, blood, or plasma. Such known screening methods do not, however, enable the health care professional reviewing the lab result to determine whether the subject is non-compliant with a prescribed drug regimen. Couto et al. (2009; J Opioid Manag.) discloses the use of an algorithm applied to urine drug screening to assess adherence to an oxycontin regimen.

SUMMARY OF THE INVENTION

In various embodiments, the present invention relates to methods for detecting or monitoring a subject's non-compliance with a prescribed drug regimen, wherein the drug comprises oxycodone, controlled-release oxycodone (OXYCONTIN®), or a metabolite of oxycodone.

The invention provides a method of reducing a risk of drug misuse in a subject comprising: determining a prescribed daily dose of a drug in the subject, wherein the drug comprises oxycodone, controlled-release oxycodone or a metabolite of oxycodone; measuring a concentration of the drug in urine of the subject; measuring parameters associated with the subject consisting of urine specific gravity, urine pH and subject lean body mass; calculating a normalized value (N) as a function of (i) the drug concentration and (ii) the parameters using the following equation: $[N] = [C] \times ((SG_N-1.00)/(SG-1.00) \times (LBW/122) \times (pH/8.50)^{0.56}$, where [C] is the raw drug concentration in ng/mL, $SG_N$ is the normalized urine specific gravity, SG is the specific gravity of the urine, LBW is the lean body weight of the subject, and pH is the pH of the urine; comparing the normalized value to a normalized mean or median estimate corresponding to the prescribed daily dose of the drug; identifying the subject at high risk of drug misuse if the normalized drug concentration falls outside the normalized mean or median estimate corresponding to the prescribed daily dose of the drug; and thereafter altering the daily dose of the drug prescribed to the subject.

These and other embodiments of the invention will be disclosed in further detail herein below.

BRIEF DESCRIPTION OF THE DRAWINGS

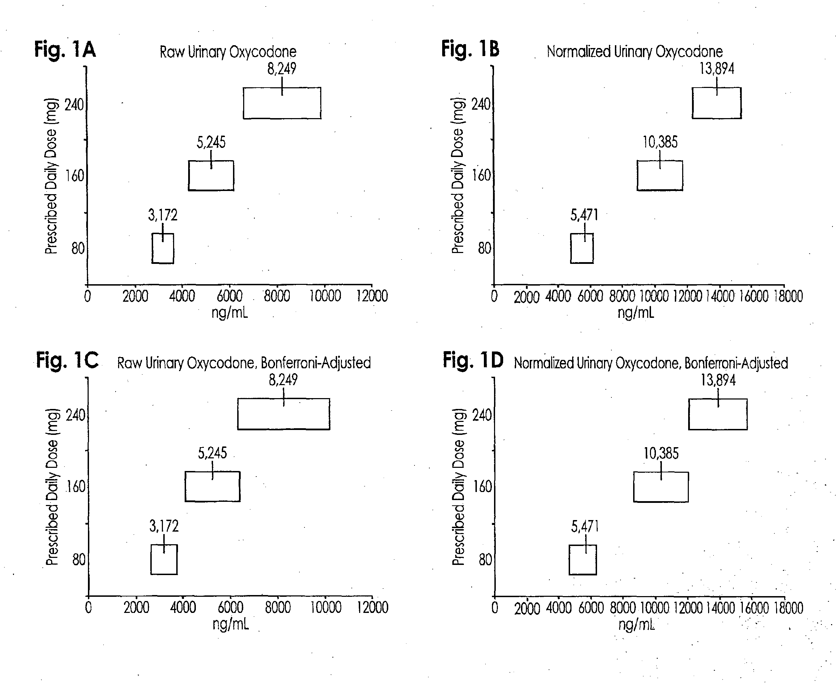

FIG. 1 shows raw (FIGs. 1A, 1C) and normalized (FIGs. 1B, 1D) median reference urine oxycodone estimates and corresponding 95% confidence intervals for each median estimate.

DETAILED DESCRIPTION OF THE INVENTION

While the present invention is capable of being embodied in various forms, the description below of several embodiments is made with the understanding that the present disclosure is to be considered as an exemplification of the invention, and is not intended to limit the invention to the specific embodiments illustrated. Headings are provided for convenience only and are not to be construed to limit the invention in any manner. Embodiments illustrated under any heading may be combined with embodiments illustrated under any other heading.

The use of numerical values in the various quantitative values specified in this application, unless expressly indicated otherwise, are stated as approximations as though the minimum and maximum values within the stated ranges were both preceded by the word "about". Also, the disclosure of ranges is intended as a continuous range including every value between the minimum and maximum values recited as well as any ranges that can be formed by such values. Also disclosed herein are any and all ratios (and ranges of any such ratios) that can be formed by dividing a disclosed numeric value into any other disclosed numeric value. Accordingly, the skilled person will appreciate that many such ratios, ranges, and ranges of ratios can be unambiguously derived from the numerical values presented herein and in all instances such ratios, ranges, and ranges of ratios represent various embodiments of the present invention.

**Therapeutic Regimens**

The method may be used in detecting non-compliance with a prescribed opioid regimen in a subject. The term "non-compliance" as used herein refers to any substantial deviation from a course of treatment that has been prescribed by a physician, nurse, nurse practitioner, physician's assistant, or other health care professional. A substantial deviation from a course of treatment may include any intentional or unintentional behavior by the subject that increases or decreases the amount, timing or frequency of opioid ingested compared to the prescribed therapy. Non-limiting examples of substantial deviations from a course of treatment include: taking more of the opioid than prescribed, taking less of the opioid than prescribed, taking the opioid more often than prescribed, taking the opioid less often than prescribed, intentionally diverting at least a portion of the prescribed opioid, unintentionally diverting at least a portion of the prescribed opioid, etc. For example, a subject substantially deviates from a course of treatment by taking about 5% to about 1000% of the prescribed daily dose or prescribed drug regimen, for example about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 105%, about 110%, about 115%, about 120%, about 125%, about 150%, about 175%, about 200%, about 225%, about 250%, about 275%, about 300%, about 350%, about 400%, about 450%, about 500%, about 550%, about 600%, about 650%, about 700%, about 750%, about 800%, about 850%, about 900%, about 950%, or about 1000% of the prescribed drug regimen. A subject may also substantially deviate from a course of treatment by taking about 5% to about 1000% more or less than the prescribed dose, for example about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 100%, about 125%, about 150%, about 175%, about 200%, about 225%, about 250%, about 275%, about 300%, about 350%, about 400%, about 450%, about 500%, about 550%, about 600%, about 650%, about 700%, about 750%, about 800%, about 850%, about 900%, about 950%, or about 1000% less than the prescribed dose. A subject may also substantially deviate from a course of treatment by, for example, taking the prescribed dose of an opioid about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 100%, about 125%, about 150%, about 175%, about 200%, about 225%, about 250%, about 275%, about 300%, 350%, 400%, 450%, 500%, 550%, 600%, about 650%, 700%, 750%, 800%, 850%, 900%, or about 1000% more often or less often than specified in the course of treatment or prescribed in the drug regimen.

The term "daily dose" or "prescribed daily dose" as used herein refers to any periodic administration of a drug to the subject over a given period of time, for example per hour, per day, per every other day, per week, per month, per year, etc. Preferably the daily dose or prescribed daily dose is the amount of the drug prescribed to a subject in any 24-hour period. The drug may be administered according to any method known in the art including, for example, orally, intravenously, topically, transdermally, subcutaneously, rectally, etc. The prescribed daily dose of the drug may be approved by the Food & Drug Administration ("FDA") for a given indication. In the alternative, a daily dose or a prescribed daily dose may be an unapproved or "off-label" use for a drug for which FDA has approved other indications. As a non-limiting example, FDA has approved oxycodone HCl controlled-release tablets (OXYCONTIN®) for use in the management of moderate to severe pain in 10 mg, 15 mg, 20 mg, 30 mg, 40 mg, 60 mg, 80 mg, 160 mg tablets. Any use of oxycodone HCl controlled-release tablets (OXYCONTIN®) other than to manage moderate to severe pain is an "off-label" use.

The method according to the present invention involves the step of determining a prescribed dose of a drug. The term "determining a prescribed dose" as used herein refers to any method known to those in the art to ascertain, discover, deduce, or otherwise learn the dose of a particular drug that has been prescribed to the subject. Non-limiting examples include subject interview, consultation with the subject's medical history, consultation with another health care professional familiar with the subject, consultation with a medical record associated with the subject, etc.

The term "drug" as used herein refers to an active pharmaceutical ingredient ("API") and its metabolites, decomposition products, enantiomers, diastereomers, derivatives, etc.

The drug comprises oxycodone, a metabolite thereof, or a controlled-release formulation thereof.

The method may be used to confirm a subject's non-adherence to a chronic opioid therapy ("COT"). The term "chronic opioid therapy" as used herein refers to any short-term, mid-term, or long-term treatment regimen comprising at least one opioid. As a non-limiting example, a subject suffering chronic pain may ingest a daily dose of oxycodone to relieve persistent pain resulting from trauma, chronic conditions, etc. COT is generally prescribed to a subject in need of such therapy; subjects on COT are typically monitored periodically by a health care professional for addiction, tolerance, or other common outcomes associated with COT. The method according to the present invention may assist a health care professional in confirming a subject's adherence or non-adherence to a COT regimen.

Subjects on COT sometimes develop an addiction to the prescribed opioid. Studies have shown that a subject on COT is more likely to develop an addiction to a prescribed opioid when he or she has a history of aberrant drug-related behavior, or is at high risk of aberrant drug-related behavior. The term "aberrant drug-related behavior" as used herein refers to any behavioral, genetic, social, or other characteristic of the subject that tends to predispose the subject to development of an addiction for an opioid. Non-limiting examples of such risk factors include a history of drug abuse, a history of opioid abuse, a history of non-opioid drug abuse, a history of alcohol abuse, a history of substance abuse, a history of prescription drug abuse, a low tolerance to pain, a high rate of opioid metabolism, a history of purposeful over-sedation, negative mood changes, intoxicated appearance, an increased frequency of appearing unkempt or impaired, a history of auto or other accidents, frequent early renewals of prescription medications, a history of or attempts to increasing dose without authorization, reports of lost or stolen medications, a history of contemporaneously obtaining prescriptions from more than one doctor, a history of altering the route of administering drugs, a history of using pain relief medications in response to stressful situations, insistence on certain medications, a history of contact with street drug culture, a history of alcohol abuse, a history of illicit drug abuse, a history of hoarding or stockpiling medications, a history of police arrest, instances of abuse or violence, a history of visiting health care professionals without an appointment, a history of consuming medications in excess of the prescribed dose, multiple drug allergies and/or intolerances, frequent office calls and visits, a genetic mutation that up-regulates or down-regulates production of drug metabolizing enzymes, a reduced-function CYP2D6 allele, and/or a non-functional CYP2D6 allele.

The present method may assist a health care professional in assessing a risk that a subject is misusing a prescribed drug. For example, a method of the disclosure comprising measuring a fluid drug concentration, normalizing the fluid drug concentration, and comparing said normalized drug concentration to an expected concentration range or normalized reference value and optionally to upper and lower confidence intervals associated with said normalized reference value to calculate a probability that the subject has misused the prescribed drug. A healthcare worker can intervene (e.g. via counseling, modifying the subject's regiment/dose, etc.) in the subject's misuse on the basis of the risk assessment.

**Sample Measurement**

The method according to the present invention includes measuring a concentration of the drug in urine of a subject, wherein the drug comprises oxycodone, controlled-release oxycodone or a metabolite of oxycodone.

Determining the amount of a drug in fluid of the subject may be accomplished by use of any method known to those skilled in the art. Non-limiting examples for determining the amount of a drug in fluid of a subject include fluorescence polarization immunoassay ("FPIA", Abbott Diagnostics), mass spectrometry (MS), gas chromatography-mass spectrometry (GC-MS-MS), liquid chromatography-mass spectrometry (LC-MS-MS), and the like. In one embodiment, LC-MS-MS methods known to those skilled in the art are used to determine a raw level, amount, or concentration of a drug in urine of the subject. In one embodiment, a raw level or concentration of a drug in fluid of a subject is measured and reported as a ratio, percent, or in relationship to the amount of fluid. The amount of fluid may be expressed as a unit volume, for example, in L, mL, $\mu$L, pL, ounce, etc. The raw amount of a drug in fluid of a subject may be expressed as an absolute level or value, for example, in g, mg, $\mu$g, ng, pg, etc.

In one embodiment, the level, concentration, or amount of a drug determined in fluid of a subject is normalized. The term "normalized" as used herein refers to a level or concentration of a drug that has been adjusted to correct for one or more parameters associated with the subject. Such a parameter may include, for example, a characteristic of the subject, a genetic trait of the subject, a behavioral predisposition of the subject, a measurable or quantifiable property associated with the subject, and the like. For example, a small percentage of the U.S. population feature variations in the cytochrome p450 allele. Broadly, an individual may have a normal CYP2D6 allele, a reduced-function CYP2D6 allele, or a non-functional CYP2D6 allele. As a result, some individuals express more or less (or no) CYP2D6 enzyme, which is an important factor in the metabolism of certain drugs. An individual that expresses more CYP2D6 enzyme is therefore expected to have a lower concentration of certain drugs in fluid measurements compared to individuals with normal levels of CYP2D6 enzymes. Normalizing measurements to account for such variations allows for more accurate comparisons. The present invention involves measuring parameters associated with the subject consisting of urine pH, urine specific gravity and subject lean body mass. Parameters may be measured by any means known in the art. For example, sample fluid pH may be measured using a pH meter, litmus paper, test strips, etc.

Also disclosed herein the raw drug concentration measured in fluid of the subject is normalized as a function of sample fluid specific gravity according to the following equation:

$$[N] = [C] \; x \; ((SG_N\text{-}1.00)/(SG\text{-}1.00)), \qquad\qquad (I)$$

where [N] is the normalized drug level, [C] is the raw drug concentration in ng/mL, SGN is the normalized fluid specific gravity, and SG is the sample fluid specific gravity.

Also disclosed herein an estimated plasma concentration is determined from a raw drug concentration level as a function of sample fluid specific gravity, the subject's lean body weight, and the sample fluid's pH according to the following equation:

$$[ETPC] = \{ \; 0.58x([C] \, x \, ((SG_N\text{-}1.00)/(SG\text{-}1.00)\} / \{(LBW/122) \, x \, (154857 \, x \, (pH\text{-}5.06))\}, \qquad (II)$$

where [ETPC] is the normalized estimated plasma concentration, [C] is the raw drug concentration in ng/mL, SGN is the normalized fluid specific gravity, SG is the specific gravity of the sample fluid, pH is the pH of the sample fluid, and LBW is the subject's lean body weight.

Also disclosed herein a normalized drug level is determined from a raw drug concentration as a function of the specific gravity of the sample fluid and the subject's weight according to the following equation:

$$[N] = [C] \; x \; ((SG_N\text{-}1.00)/(SG\text{-}1.00)) \; x \; (W/154), \qquad\qquad (III)$$

where [N] is the normalized drug level, [C] is the raw drug concentration in ng/mL, SGN is the normalized fluid specific gravity, SG is the specific gravity of the sample, and W is the weight of the subject in pounds.

In the invention, the normalized drug level is determined from a raw drug concentration as a function of the specific gravity of the sample, the subject's lean body weight, and the pH of the sample according to the following equation:

$$[N] = [C] \; x \; ((SG_N\text{-}1.00)/(SG\text{-}1.00)) \; x \; (LBW/122) \; x \; (pH/8.5)^{56}, \qquad (IV)$$

where [N] is the normalized drug level, [C] is the raw drug concentration in ng/mL, SGN is the normalized fluid specific gravity, SG is the specific gravity of the sample, LBW is the lean body weight of the subject, and pH is the pH of the sample.

In the invention, the normalized drug level is determined from a raw drug concentration as a function of the specific gravity of the sample, the subject's lean body weight, and the pH of the sample according to the following equation:

$$[N] = [C] \; x \; ((SG_N\text{-}1.00)/(SG\text{-}1.00) \; x \; (LBW/122) \, x \, (pH/8.50)^{56} \qquad\qquad (V)$$

where [N] is the normalized drug level, [C] is the raw drug concentration in ng/mL, SGN is the normalized fluid specific gravity, SG is the specific gravity of the sample, LBW is the lean body weight of the subject in pounds, and pH is the pH of the sample. A normalized oxycodone level may be determined from a raw urine oxycodone level as a function of the specific gravity of the urine sample, the subject's lean body weight, and the pH of the urine sample according to Equation (V) where SGN is equal to 1.03. Also disclosed herein, a normalized oxycodone level is determined from a raw urine oxycodone level as a function of the specific gravity of the urine sample, the subject's lean body weight, and the pH of the urine sample according to Equation (V), wherein the raw urine oxycodone level is measured by GC-MS-MS or LC-MS-MS and represents the total raw urine concentration of oxycodone, oxymorphone, and noroxycodone.

Also disclosed herein, raw drug concentrations are normalized as a function of the concentration of creatinine in the sample fluid according to the following equation:

$$[N] = [C] \; x \; ((CR_N\text{-}1.00)/(CR\text{-}1.00), \qquad\qquad (VI)$$

where [N] is the normalized drug level, [C] is the raw drug concentration in ng/mL, CRN is the normalized concentration of creatinine in fluid of the same type as the sample fluid obtained from the subject, and CR is the raw concentration of creatinine in the sample obtained from the subject.

"Lean body weight" or "LBW" as used herein refers to a subject's weight excluding the weight of the subject's body fat. Lean body weight may be calculated by any of the following equations:

$$LBW = Body\ Weight\ - Fat\ Weight \tag{VII}$$

$$LBW = (Body\ Weight)\ x\ (Body\ Weight\ \%)/100 \tag{VIII}$$

$$LBW = (Subject\ Body\ Weight)\ x\ [1 - (Body\ Fat\ \%)/100] \tag{IX}$$

Alternatively, lean body weight or LBW may be estimated by any method known in the art. Non-limiting examples of estimations of LBW include:

$$LBW_{men} = (0.32810\ x\ (body\ weight_{kg})) + (0.33929\ x\ (height_{cm})) - 29.5336 \tag{X}$$

$$LBW_{men} = \frac{2.2\ x\ (2.447 - (0.09516\ x\ age_{yrs}) + (0.2728\ x\ height_{cm}) + (0.1528\ x\ weight_{lbs})}{0.73} \tag{XI}$$

$$LBW_{women} = (0.29569\ x\ (body\ weight_{kg})) + (0.41813\ x\ (height_{cm})) - 43.2933 \tag{XII}$$

$$LBW_{women} = \{2.2\ x\ (-2.097 + (-2.097 + (0.2715\ x\ height_{cm}) + (0.1121\ x\ weight_{lbs})\}/0.73 \tag{XIII}$$

Also disclosed herein, the level or concentration of drug in fluid of the subject is normalized as a function of the subject's body surface area. A non-limiting example of one method to estimate a subject's body surface area in square meters is by the equation:

$$BSA = (height_{cm}\ x\ weight_{kg}/3600)^{0.5} \tag{XIV}$$

In one embodiment, the concentration or level of drug in fluid of the subject is a steady state concentration or level. The term "steady state" as used herein refers to an equilibrium level or concentration of a drug obtained at the end of a certain number of administrations (e.g. 1 to about 5). Steady state is achieved when the concentration or level of the drug will remain substantially constant if the dose and the frequency of administrations remain substantially constant.

**Normalizing Clinical Data**

The amount of drug measured or determined in fluid of a subject may be compared to a reference level of the same drug. The term "reference level" as used herein refers to a standard amount of a drug expected to be present in fluid of a subject that has been administered a given dose of the drug. A reference level may be a steady state level of a drug or may be a single point-in- time level of the drug. Generally, the reference level for a drug in one type of fluid may not be the same as the reference level for the same drug in a different type of fluid. For example, a drug that is quickly excreted through urine may have a higher reference level in urine than in blood or plasma. In contrast, a drug that is slowly excreted may have a higher reference level in blood or plasma than in urine.

A reference level for a given drug may be expressed in various ways. Reference levels may be expressed as a mean, median, average, or weighted average reference value. Optionally, confidence intervals for a given reference, value may be established by any suitable statistical methods or models. For example, a raw reference value for oxycodone measured in urine may be 3,172 ng/mL for a daily dose of 80 mg. Confidence intervals provide health care professionals useful data for determining whether a given subject's oxycodone urine level correlates well with the subject's prescribed dose. Thus, 95% confidence levels for the above example of, for example, 2,730-3,613 ng/mL provide the health care professional upper and lower boundaries. The 95% upper and lower confidence intervals represent the range of oxycodone urine levels in which samples from 95% of the population compliant with that prescribed daily dose are expected to fall. Other confidence intervals may be established for a given reference value. Non-limiting examples of confidence intervals include about 99.9%, 99.5%, 99%, 98%, 97%, 96%, 95%, 94%, 93%, 92%, 91%, 90%, 85%, 80%, 75%, 70%, 65%, 60%, 55%, 50%, 45%, 40%, 35%, 30%, 25%, 20%, 15%, 10%, and 5%. Reference values and associated confidence intervals may be established for a plurality of prescribed daily doses of a drug. Confidence intervals for each prescribed daily dose may not substantially overlap. Each prescribed daily dose may differ from other daily doses by at least 50%, at least 75%, at least 100% or at least 150%.

Confidence intervals for each daily dose of a drug do not overlap. The phrase "do not overlap" as used in the present context means, for example, that the lower confidence interval for one daily dose of a drug does not overlap with the upper confidence interval for a second, smaller daily dose of the same drug.

Reference levels may be provided as a concentration range. The term "concentration range" as used herein refers to a continuous range of drug concentrations in fluid of a subject who has been administered a given drug. Concentration ranges are generally unique for a given drug and daily dose of that drug. Concentration ranges may be established by any suitable method known to those skilled in the art, and may be based on a single subject or a population of subjects. For example, concentration ranges may be determined based on a reference value of a drug and associated confidence intervals. As a non-limiting example, and as shown in FIG. IA, a raw concentration range for a 160 mg prescribed daily dose of oxycodone may be 4,311-6,178 ng/mL, derived from a raw reference value of 5,245 ng/mL, an associated 95% lower confidence interval of 4,311 ng/mL and an associated 95% upper confidence interval of 6,178 ng/mL. Such a concentration range may also be reported as a mean, median, or average value with an error value, for example as 5,245 $\pm$ 933.5 ng/mL for a 160 mg daily dose of oxycodone. Concentration ranges may be established for each of a plurality of daily doses of a drug. Concentration ranges for each of a plurality of daily doses of a drug may not substantially overlap.

The concentration ranges for each prescribed daily dose of a drug may not overlap. For example, the upper-bound of the concentration range of one prescribed daily dose of a drug is less than or equal to the lower-bound of the concentration range of a second, higher prescribed daily dose of the same drug. Preferably in the example described, the upper-bound of the concentration range of one prescribed daily dose of a drug is less than but not equal to the lower-bound of the concentration range of a second, higher prescribed daily dose of the same drug.

Reference levels, reference values and associated confidence intervals, and/or concentration ranges may be derived from a population of subjects. The term "a population" as used herein refers to any group or selection of subjects for which a reference level, reference value and associated confidence intervals, or concentration range is desired. One or a plurality of subjects may be assigned to a population. As used herein a "plurality of subjects" refers to two or more subjects, for example about 2 subjects, about 3 subjects, about 4 subjects, about 5 subjects, about 6 subjects, about 7 subjects, about 8 subjects, about 9 subjects, about 10 subjects, about 15 subjects, about 20 subjects, about 25 subjects, about 30 subjects, about 35 subjects, about 40 subjects, about 45 subjects, about 50 subjects, about 55 subjects, about 60 subjects, about 65 subjects, about 70 subjects, about 75 subjects, about 80 subjects, about 85 subjects, about 90 subjects, about 95 subjects, about 100 subjects, about 110 subjects, about 120 subjects, about 130 subjects, about 140 subjects, about 150 subjects, about 160 subjects, about 170 subjects, about 180 subjects, about 190 subjects, about 200 subjects, about 225 subjects, about 250 subjects, about 275 subjects, about 300 subjects, about 325 subjects, about 350 subjects, about 375 subjects, about 400 subjects, about 425 subjects, about 450 subjects, about 475 subjects, about 500 subjects, about 525 subjects, about 550 subjects, about 575 subjects, about 600 subjects, about 625 subjects, about 650 subjects, about 675 subjects, about 700 subjects, about 725 subjects, about 750 subjects, about 775 subjects, about 800 subjects, about 825 subjects, about 850 subjects, about 875 subjects, about 900 subjects, about 925 subjects, about 950 subjects, about 975 subjects, about 1000 subjects, about 1250 subjects, about 1500 subjects, about 1750 subjects, about 2000 subjects, about 2250 subjects, about 2500 subjects, about 2750 subjects, about 3000 subjects, about 3500 subjects, about 4000 subjects, about 4500 subjects, about 5000 subjects, about 5500 subjects, about 6000 subjects, about 6500 subjects, about 7000 subjects, about 7500 subjects, about 8000 subjects, about 8500 subjects, about 9000 subjects, about 9500 subjects, or about 10000 subjects. As used herein with respect to a population, the term "subject" is synonymous with the term "member" and refers to an individual that has been assigned to the population. Subpopulations may be established for a plurality of daily doses of a drug.

A plurality of subjects of a population are each prescribed the same daily dose of a drug. A plurality of subjects may be assigned to one subpopulation are each prescribed a first daily dose of a drug while a plurality of subjects assigned to a second, different subpopulation are each prescribed a second, different daily dose of a drug. IA plurality of subjects may be assigned to a population or subpopulation are each prescribed a daily dose of a drug for a time sufficient to achieve steady state. The term "time sufficient to achieve steady state" refers to the amount of time required, given the pharmacokinetics of the particular drug and the dose administered to the subject, to establish a substantially constant concentration or level of the drug assuming the dose and the frequency of administrations remain substantially constant. The time sufficient to achieve steady state may be determined from literature or other information corresponding to the drug. For example, labels or package inserts for FDA approved drugs often include information regarding typical times sufficient to achieve steady state plasma concentrations from initial dosing. Other non- limiting means to determine the time sufficient to achieve steady state include experiment, laboratory studies, analogy to similar drugs with similar absorption and excretion characteristics, etc.

Assignment of subjects to a population or subpopulation may be accomplished by any method known to those skilled in the art. For example, subjects may be assigned randomly to one of a plurality of subpopulations. Subjects are screened for one or more parameters before or after being assigned to a population. For example, subjects featuring one or more parameters that may tend to affect fluid levels of a drug may be excluded from a population, may not be assigned to a population, may be assigned to one of a plurality of subpopulations, or may be removed from a population or subpopulation during or after a data collection phase of a study. Subjects with reduced-function CYP2D6 alleles or non-functioning CYP2D6 alleles may be excluded from a population because such variants are known to affect the normal opioid metabolic rate. Subjects with reduced-function CYP2D6 alleles may be assigned to a first subpopulation, subjects with non-functioning CYP2D6 alleles are assigned to a second subpopulation, and subjects with normal CYP2D6 alleles are assigned to a third subpopulation. Other non-limiting examples of exclusion criteria include: histories of substance abuse; significant disease such as cancer; cardiac disease, autoimmune disease, neurological disease, and the like; recent illness; abnormal findings on physical examination, electrocardiogram, laboratory studies, or drug screens; recent history of prescription drug use, over-the-counter ("OTC") drug use, or herbal drug use; allergies or hypersensitivities to naltrexone, opioids, or similar compounds; recent history of use of alcohol, ingestion of grapefruit, ingestion of grapefruit juice, ingestion of caffeine, or ingestion of xanthene-containing products; and participation in other drug therapy or opioid-related clinical trial or study.

One or more parameters of a plurality of subjects in a population or subpopulation may be measured or ascertained according to the disclosure. Non-limiting examples of parameters include fluid pH, fluid specific gravity, fluid creatinine concentration, subject height, subject weight, subject age, subject body mass index, subject gender, and subject lean body mass, and subject body surface area. Parameters may include fluid specific gravity, fluid pH, subject body weight, subject gender, and subject height. Parameters may include fluid specific gravity, fluid pH, and subject lean body mass. Lean body mass may be calculated or estimated as already discussed, supra. In the invention, the parameters consist of urine specific gravity, urine pH and subject lean body mass.

Drug levels are measured in fluid of a plurality of subjects in a population. Drug levels in a subject's fluid may be measured by any suitable means known to those skilled in the art. Non-limiting examples for determining the amount of a drug in fluid of a subject include fluorescence polarization immunoassay ("FPIA", Abbott Diagnostics), mass spectrometry (MS), gas chromatography-mass spectrometry (GC-MS-MS), liquid chromatography- mass spectrometry (LC-MS-MS), and the like. In one embodiment, LC-MS-MS methods known to those skilled in the art are used to determine the amount of a drug in urine of the subject. The concentration of a drug in fluid of a subject may be measured and reported as a ratio, percent, or in relationship to the amount of fluid. The amount of fluid may be expressed as a unit volume, for example, in L, mL, $\mu$L, pL, ounce, etc. The amount of a drug in fluid of a subject may be expressed as an absolute level or value, for example, in g, mg, $\mu$g, ng, pg, etc.

In the following section, various methods and equations for normalising raw drug concentrations are disclosed. The equation used in the invention is that specified in claim 1, namely equation XIX.

The raw drug concentrations measured in fluid of a plurality of subjects in a population may be normalized as a function of sample fluid specific gravity according to the following equation:

$$[N] = [C] \; x \; ((SG_N\text{-}1.00)/(SG\text{-}1.00)), \qquad\qquad (XV)$$

where [N] is the normalized drug level, [C] is the raw drug concentration in ng/mL, SGN is the normalized fluid specific gravity, and SG is the sample fluid specific gravity.

An estimated plasma concentration may be determined from the raw drug concentration levels in a plurality of subjects in a population as a function of sample fluid specific gravity, the subject's lean body weight, and the sample fluid's pH according to the following equation:

$$[ETPC] = \{ 0.58\,x\,([C]\,x\,((SG_N\text{-}1.00)/(SG\text{-}1.00)\}/\{(LBW/122)\,x\,(154857\,x\,(pH\text{-}5.06))\}, \qquad (XVI)$$

where [ETPC] is the normalized estimated plasma concentration, [C] is the raw drug concentration in ng/mL, SGN is the normalized fluid specific gravity, SG is the specific gravity of the sample fluid, pH is the pH of the sample fluid, and LBW is the subject's lean body weight.

Normalized drug levels for a plurality of subjects in a population may be determined from raw drug concentrations as a function of the specific gravity of the sample fluid and the subject's weight according to the following equation:

$$[N] = [C] \times ((SG_N\text{-}1.00)/(SG\text{-}1.00)) \times (W/154), \qquad (XVII)$$

where [N] is the normalized drug level, [C] is the raw drug concentration in ng/mL, SGN is the normalized fluid specific gravity, SG is the specific gravity of the sample, and W is the weight of the subject in pounds.

Normalized drug levels may be determined from the raw drug concentrations of a plurality of subjects in a population as a function of the specific gravity of the sample, the subject's lean body weight, and the pH of the sample according to the following equation:

$$[N] = [C] \times ((SG_N\text{-}1.00)/(SG\text{-}1.00)) \times (LBW/122) \times (pH/8.50)^{56} \quad . \qquad (XVIII)$$

where [N] is the normalized drug level, [C] is the raw drug concentration in ng/mL, SGN is the.normalized fluid specific gravity, SG is the specific gravity of the sample, LBW is the lean body weight of the subject, and pH is the pH of the sample.

Raw drug concentrations may be measured in fluid in a plurality of subjects are normalized as a function of the specific gravity of the sample, the subject's lean body weight, and the pH of the sample according to the following equation:

$$[N] = [C] \times ((SG_N\text{-}1.00)/(SG\text{-}1.00) \times (LBW/122) \times (pH/8.50)^{56}), \qquad (XIX)$$

where [N] is the normalized drug level, [C] is the raw drug concentration in ng/mL, SGN is the normalized fluid specific gravity, SG is the specific gravity of the sample, LBW is the lean body weight of the subject in pounds, and pH is the pH of the sample. Raw oxycodone levels may be measured in urine of a plurality of subjects is normalized as a function of the specific gravity of the urine sample, the subject's lean body weight, and the pH of the urine sample according to Equation (XIX) where SGN is equal to 1.03. Raw oxycodone levels may be measured in urine of a plurality of subjects are normalized as a function of the specific gravity of the urine sample, the subject's lean body weight, and the pH of the urine sample according to Equation (XVIII), wherein the raw urine oxycodone level is measured by GC-MS-MS or LC-MS-MS and represents the total raw urine concentration of oxycodone, oxymorphone, and noroxycodone.

Normalized drug levels for a plurality of subjects in a population may be determined from raw drug concentrations as a function of the concentration of creatinine in the sample fluid according to the following equation:

$$[N] = [C] \times ((CR_N\text{-}1.00)/(CR\text{-}1.00), \qquad (XX)$$

where [N] is the normalized drug level, [C] is the raw drug concentration in ng/mL measured in fluid from a plurality of subjects, CRN is the normalized concentration of creatinine in fluid of the same type as the sample fluid obtained from the plurality of subjects, and CR is the raw concentration of creatinine in the sample obtained from a plurality of subjects.

"Lean body weight" or "LBW" as used herein refers to a subject's weight or excluding the weight of the subject's body fat. Lean body weight may be calculated by any of the following equations:

$$LBW = Body\ Weight - Fat\ Weight \qquad (XXI)$$

$$LBW = (Body\ Weight) \times (Body\ Weight\ \%)/100 \qquad (XXII)$$

$$LBW = (Subject\ Body\ Weight) \times [1 - (Body\ Fat\ \%)/100] \qquad (XXIII)$$

Alternatively, lean body weight or LBW may be estimated by any method known in the art. Non-limiting examples of estimations of LBW include:

$$LBWmen = (0.32810 \times (body\ weight_{kg})) + (0.33929 \times (height_{cm})) - 29.5336 \qquad (XXIV)$$

$$LBWmen = \frac{2.2 \times (2.447 - (0.09516 \times age_{yrs}) + (0.2728 \times height_{cm}) + (0.1528 \times weight_{lbs})}{0.73} \qquad (XXV)$$

$$LBW_{women} = (0.29569 \times (body\ weight_{kg})) + (0.41813 \times (height_{cm}) - 43.2933 \qquad (XXVI)$$

$$LBW_{women} = \frac{2.2 \times (-2.097 + (-2.097 + (0.2715 \times height_{cm}) + (0.1121 \times weight_{lbs})}{0.73} \qquad (XXVII)$$

The level or concentration of drug in fluid of a plurality of subjects may be normalized as a function of the subject's body surface area. A non-limiting example of one means to estimate a subject's body surface area in square meters is by the equation:

$$BSA = (height_{cm} \times weight_{kg}/3600)^{0.5} \qquad (XXVIII)$$

The concentration or level of drug in fluid of a plurality of subjects may be a steady state concentration or level.

A drug reference value for a population may be determined by aggregating drug concentrations or drug levels for a plurality of subjects assigned to a population. A normalized drug reference value for a population may be determined from aggregation of a plurality of normalized drug concentrations or drug levels of a plurality of subjects assigned to the population. Aggregation of drug concentrations, drug levels, normalized drug concentrations, and/or normalized drug levels may be accomplished by any suitable method known to those skilled in the art. For example, a drug level for a population may be determined as the mean, median, average, or weighted average of a plurality of drug levels measured in a plurality of subjects assigned to the population. Confidence intervals for a reference drug value may be determined for a population. Non-limiting examples of confidence intervals include 99.9%, 99.5%, 99%, 98%, 97%, 96%, 95%, 94%, 93%, 92%, 91%, 90%, 85%, 80%, 75%, 70%, 65%, 60%, 55%, 50%, 45%, 40%, 35%, 30%, 25%, 20%, 15%, 10%, and 5%. Reference values and associated confidence intervals for a population may be established for a plurality of daily doses of a drug. Confidence intervals for each daily dose may not substantially overlap. Confidence intervals for each daily dose of a drug may not overlap.

Reference levels for a population are provided as a normalized concentration range. Normalized concentration ranges for a population may be established by any suitable method known to those skilled in the art. For example, normalized concentration ranges may be determined based on a normalized reference value of a drug for a population and the associated confidence intervals. As a non-limiting example, as shown in FIG. 1B, a normalized concentration range for a population of subjects prescribed a 160 mg daily dose of oxycodone may be 9,004-11,765; derived from a normalized reference value of 10,385 and an associated 95% lower confidence interval of 9004 and an associated 95% upper confidence interval of 11,765. Such a normalized concentration range may also be reported as a mean, median, or average value with an error value, for example as 9,004 ± 1,381 for a prescribed 160 mg daily dose of oxycodone. Normalized concentration ranges for a population may be established for each of a plurality of prescribed daily doses of a drug. Normalized concentration ranges for each of a plurality of prescribed daily doses of a drug may not substantially overlap. Normalized concentration ranges for each of a plurality of prescribed daily doses of a drug may not overlap.

A normalized concentration range may be determined from the aggregation of a plurality of normalized fluid drug levels. For example, a normalized concentration range for a given daily dose of oxycodone is determined as the average of a plurality of oxycodone drug levels of subjects in a population. Standard statistical methods known to those skilled in the art may be used to determine a concentration range, for example, using standard deviations, Bonett-Price analysis, or Bonferroni adjustments for multiple comparisons.

Normalization of concentration ranges can be used to distinguish concentration ranges for a plurality of daily doses of a drug. For example, FIG. I shows median urinary concentrations (measured by LC-MS-MS) and associated 95% confidence intervals for three daily doses of controlled-release oxycodone (OXYCONTIN®): 80 mg, I 60 mg, and 240 mg. The data in FIG. IA have not been normalized nor adjusted by Bonferroni adjustments for multiple comparisons. The data in FIG. IB have been normalized as a function of urine pH, urine specific gravity, and lean body weight and shows greater distinction between the three daily doses. The data in FIG. I C have not been normalized but has been adjusted by Bonferroni adjustments for multiple comparisons. In this case the Bonett-Price 95% confidence intervals overlap: the upper confidence interval for a prescribed I60 mg daily dose overlaps with the lower confidence interval for 240 mg. When the same data are normalized as a function of urine pH, urine specific gravity, and lean body mass and then adjusted by Bonferroni adjustments for multiple comparisons (FIG. ID), Bonett-Price 95% confidence intervals for the three daily doses no longer overlap.

A normalized reference drug level and associated confidence intervals may be determined from the aggregation of a plurality of normalized fluid drug levels. For example, a normalized reference drug level for a given daily dose of oxycodone is determined as the average of a plurality of fluid oxycodone levels of subjects in a population. Standard statistical methods may then be used to determine upper and lower confidence intervals, for example, using standard deviations or methods according to Bonett and Price, and optionally Bonferroni adjustments for multiple comparisons.

Reference drug median estimates and associated confidence intervals may be established according to the methods of Bonett and Price, for example as described in Psychological Methods, vol. 7, pp. 370-383 (2002), J. Stat. Comput. Simul., vol. 68, pp. 295-305 (2001), and J. Stat. Comput. Simul., vol. 72, pp. 119-124 (2002).

Bonferroni adjustments, also sometimes referred to as the Bonferroni method, allow multiple comparisons to be made while maintaining a satisfactory overall confidence coefficient.

**Comparison to a Reference Level**

A subject's non-compliance with a prescribed treatment protocol or treatment regimen may be confirmed by comparing the subject's fluid drug level to a reference drug level. The method may be used in combination with any other method known to those skilled in the art for detecting a subject's potential non-compliance with a prescribed treatment protocol. Non-limiting examples of such methods include: interviews with the subject, fluid testing for the presence or absence of detectable levels of a drug, observation of the subject's behavior, appreciating reports of diversion of the subject's prescribed drug to others, etc.

The subject's fluid drug level may be normalized for one or more parameters. The reference drug level may be normalized for one or more parameters. The subject's fluid drug level and the reference drug level may be normalized for one or more parameters. The subject's fluid drug level and the reference drug level may both be normalized as a function of the same set of one or more parameters. For example, the subject's fluid drug level may be normalized as a function of fluid pH, fluid specific gravity, and subject body weight; and the reference drug level is normalized as a function of fluid pH, fluid specific gravity, and subject body weight.

A subject's non-compliance may be confirmed if the subject's fluid drug level or concentration falls outside the reference drug level, reference drug value and associated confidence intervals, and/or reference drug concentration range for the daily dose prescribed to the subject. The subject's fluid level or concentration may be normalized for one or more parameters associated with the subject. The reference drug level, reference drug value and associated confidence intervals, and/or reference drug concentration range may be normalized for one or more parameters associated with the members of the population. The subject's fluid drug level or concentration may be normalized and the reference drug level, reference drug value and associated confidence intervals, and/or reference drug concentration range is also normalized. The subject's fluid drug level may be a steady state fluid drug level. The reference drug level, reference drug value and associated confidence intervals, and/or reference drug concentration range may be determined from steady state fluid drug levels for a plurality of members of the relevant population.

All fluid drug levels measured in the subject and the plurality of members of the relevant population may be steady state fluid drug levels. The fluid of the subject is the same type of fluid as the fluid of the members of the relevant population. The fluid is urine.

A method according to the present disclosure may be used to identify or determine a subject's non-compliance with a prescribed treatment protocol. The method may comprise measuring a drug concentration in fluid of the subject; measuring one or more parameters associated with the subject; calculating a normalized drug concentration value for the subject as a function of the drug concentration and the one or more measured parameters; developing, in a population that does not include the subject, a normalized reference drug concentration range corresponding to the prescribed treatment protocol; comparing the normalized drug concentration value of the subject to the normalized reference drug concentration range; and determining that the subject is non-compliant if the normalized drug concentration of the subject falls outside of the normalized reference drug concentration range.

Also disclosed herein is a method used to identify a subject at high risk of oxycodone misuse. The method may comprise determining a prescribed daily dose of oxycodone in the subject; measuring a concentration of oxycodone in urine of the subject; measuring one or more parameters in the subject and/or the urine of the subject; calculating a normalized oxycodone concentration value as a function of the oxycodone concentration and the one or more parameter; comparing the normalized oxycodone concentration value to a normalized mean or median estimate corresponding to the prescribed daily dose of oxycodone; and identifying the subject at high risk of oxycodone misuse if the normalized oxycodone concentration value falls outside upper and lower confidence levels corresponding to the prescribed daily dose of oxycodone.

In an embodiment of the invention, the normalized mean or median estimate and associated upper and lower confidence levels corresponding to the prescribed daily dose of oxycodone are determined by measuring a steady state drug concentration in urine of each of a plurality of members of a population; measuring one or more parameters in each member; normalizing the drug concentration for each member as a function of the one or more parameters; determining the normalized mean or median estimate corresponding to the prescribed daily dose of the drug from the normalized drug concentrations of the population; and determining upper and lower confidence intervals for the normalized mean or median estimate.

The invention provides a method of reducing a risk of drug misuse in a subject comprising: determining a prescribed daily dose of a drug in the subject; measuring a concentration of the drug in urine of the subject; measuring parameters associated with the subject consisting of urine specific gravity, urine pH and subject lean body mass; calculating a normalized value (N) as a function of (i) the drug concentration and (ii) the parameters using the following equation: $[N] = [C] \times ((SG_N-1.00)/(SG-1.00) \times (LBW/122) \times (pH/8.50)^{0.56}$, where $[C]$ is the raw drug concentration in ng/mL, $SG_N$ is the normalized urine specific gravity, SG is the specific gravity of the urine, LBW is the lean body weight of the subject, and pH is the pH of the urine; comparing the normalized value to a normalized mean or median estimate corresponding to the prescribed daily dose of the drug; identifying the subject at high risk of drug misuse if the normalized drug concentration value falls outside the normalized mean or median estimate corresponding to the prescribed daily dose of the drug; and thereafter altering the daily dose of the drug prescribed to the subject. The drug comprises oxycodone, controlled-release oxycodone or a metabolite of oxycodone.

A method disclosed herein may be used to confirm a subject's non-adherence to a chronic opioid therapy (COT) regimen, e.g. comprising prescribing to the subject a chronic opioid therapy regimen, said regimen comprising a daily dose of an opioid; determining, after a time sufficient to achieve steady state, a normalized opioid concentration value in the subject; developing, in a secondary population that does not include the subject, a normalized mean or median opioid estimate and associated upper and lower confidence intervals corresponding to the prescribed daily dose of the opioid; comparing the normalized opioid concentration value in the subject to the normalized mean or median opioid estimate corresponding to the prescribed daily dose of the opioid; and determining the subject's non-adherence to the chronic opioid therapy regimen if the normalized opioid concentration value in the subject falls outside the confidence intervals corresponding to the prescribed daily dose of the opioid. The opioid may comprise oxycodone, controlled-release oxycodone, a metabolite of oxycodone, or combinations thereof. The subject has a history of or is at high risk of developing aberrant drug-related behavior.

The normalized opioid concentration in the subject may be determined by measuring a steady state opioid concentration in urine of the subject; measuring one or more parameters in the subject and/or urine of the subject; and calculating the normalized opioid concentration in the subject as a function of the one or more parameters measured in the subject and/or urine of the subject. The one or more parameters may be selected from the group consisting of urine pH, urine specific gravity, urine creatinine concentration, subject height, subject weight, subject age, subject body mass index, subject gender, subject lean body mass, and subject body surface area.

A method according to the present disclosure may be used to identify a risk of drug misuse in a population. Also disclosed herein is a method comprising assigning a plurality of subjects to a first population; administering to the plurality of subjects a daily dose of a drug; determining a level of said drug in fluid of the plurality of subjects; measuring one or more parameters associated with the plurality of subjects; normalizing said levels of said drug as a function of at least one of said parameters; developing, in a second population that does not include said plurality of subjects, a normalized reference drug level and associated confidence intervals corresponding to the daily dose of the drug; comparing the normalized levels of said drug in the first population to the normalized reference drug level and associated confidence intervals; and identifying a risk of drug misuse in a first population if the normalized levels of said drug in the first population fall outside the associated confidence intervals corresponding to the daily dose of the drug.

Also disclosed herein is a method which provides a probability that a subject is non-compliant with a prescribed drug regimen. A raw drug level measured in fluid obtained from a subject may be normalized as a function of one or more parameters associated with the subject. A probability that the subject is non-compliant with a prescribed drug regimen is then determined by comparing the normalized drug level to at least one of: a normalized reference drug level associated with the prescribed drug regimen, upper and lower confidence intervals associated with said normalized reference drug level, and/or a normalized concentration range associated with the prescribed drug regimen.

Also disclosed herein is a refined probability that the subject is non-compliant with a prescribed drug regimen results from the combination of the probability that the subject is non-compliant (e.g., determined by comparing a normalized drug level in fluid of the subject to at least one of a normalized reference drug level associated with the prescribed drug regimen, upper and lower confidence intervals associated with said normalized reference drug level, and/or a normalized concentration range associated with the prescribed drug regimen) with a pretest probability. The phrase "pretest" as used herein refers to any assessment tool known to those skilled in the art other than diagnostic testing of fluid obtained from a subject as set forth herein that tends to predict or demonstrate a subject's compliance with a prescribed drug regimen. The pre-test probability may be derived from at least one of: administration of one or more questionnaires, administration of one or more standard risk screening instruments, appreciation of one or more aberrant drug behaviors, conducting of one or more interviews with friends of the subject, conducting of one or more interviews with relatives of the subject, conducting of one or more interviews with acquaintances of the subject, and the like. The refined probability that a subject is non-compliant may result from the multiplication of the pretest probability with the probability determined from comparison of a normalized drug level in fluid of the subject to at least one of a normalized reference drug level associated with the prescribed drug regimen, upper and lower confidence intervals associated with said normalized reference drug level, and/or a normalized concentration range associated with the prescribed drug regimen.

EXAMPLES

The following examples are for illustrative purposes only and are not to be construed as limiting the scope of the invention in any respect whatsoever.

EXAMPLE 1

Thirty-six healthy adult, non-smoking subjects, 18-50 years of age, with body mass index between 18-50 years of age with body mass index between 18 and 32 kg/m3 were studied in a single-group, multiple dose study of 80, 160, and 240 mg daily dose controlled-release oxycodone (OXYCONTIN®). Fifteen of the subjects were female; 21 were male. Women were required to have a negative urine pregnancy test before the study initiation as well as to use a medically accepted method of contraception throughout the duration of the study. All participants were screened for phenotypic variation of the CYP2D6 enzymes using a commercially available screening test (PGXL Laboratories, Louisville, KY), and ultlarapid, rapid, and poor metabolizers were excluded from the study. Other exclusions included individuals with histories of substance abuse, significant disease, recent illness, or abnormal finding on physical examination, electrocardiogram, laboratory studies, or drug screens. Additionally, those with recent histories of prescription, over the counter, and herbal drug use were excluded. The subjects with allergies or hypersensitivities to naltrexone, oxycodone, other opioids, or similar compounds were ineligible to participate. These subjects were forbidden to use alcohol, ingest grapefruit, grapefruit juice, caffeine, or xanthene-containing products 48 hours before dosing and during the dosing periods.

Each subject received naltrexone blockade throughout the study (50 mg daily naltrexone dosing was initiated 12 hours before oxycodone administration and continued through day 4) according to the study protocol. The subjects ranged in age from 18 to 50 years (mean = 23.58) and averaged 68 inches in height and 159 pounds in weight. Thirty-two participants were Caucasian.

The subjects were randomized to one of the three dosages of controlled-release oxycodone: 80, 160, or 240 mg/d dosed every 12 hours through day 4. On day 2, two presteady-state urine samples were collected every 12 hours. The half-life of controlled-release oxycodone is 4.5 hours, and most patients will reach steady state after 4-5 half lives of a drug, leaving most patients taking controlled- release oxycodone at steady state before day 3. Previous pharmacokinetic studies have confirmed that upon repeated dosing of controlled-release oxycodone, patients achieve steady-state levels within 24-36 hours. Beginning on day 3 at midnight, urine samples of all subjects were collected through 23:59 on day 4, while subjects were at steady state. A total of 373 urine samples were collected while the subjects were in steady state for each dose of controlled-release oxycodone. In addition, pK blood and oral fluid samples were collected three times on days 3 and 4. Laboratory, medication, physical examination, and adverse event findings were collected in the event of early termination or at the follow-up visit. Raw oxycodone levels in the urine samples were determined by LC-MS-MS and included the total detectable measurements for noroxycodone, oxymorphone, and oxycodone in the urine.

An analysis of medians was conducted for each of the daily dosage groupings using Bonett-Price confidence intervals for both raw and normalized drug levels. An analysis of medians, rather than means in this instance, was more appropriate because (a) the distribution of values in this relatively small sample appears skewed at each dose level; (b) no information as to the true population distribution of drug levels was available, and (c) the analysis of medians is robust to almost any type of non-normality that would likely be encountered in practice. The Bonett-Price confidence interval method was chosen in particular because of its superior performance in simulation experiments of small samples. For comparison purposes, and to establish even more conservative estimates, a Bonferroni adjustment was applied to the confidence intervals.

**TABLE 1.**

| A | B | C | D | E \| F | |
|---|---|---|---|---|---|
| Prescribed Daily Dose (mg) | Median Urinary Drug Level | 95% Lower Confidence | 95% Upper Confidence Interval | 95% Lower Confidence | 95% Upper Confidence |
| Raw Data (ng/mL) | | | | | |
| 80 | 3,172 | 2,730 | 3,613 | 2,632 | 3,711 |
| 160 | 5,245 | 4,311 | 6,178 | 4,105 | 6,384 |
| 240 | 8,249 | 6,647 | 9,851 | 6,292 | 10,206 |
| Normalized Data | | | | | |
| 80 | 5,471 | 4,796 | 6,147 | 4,646 | 6,296 |
| 160 | 10,385 | 9,004 | 11,765 | 8,699 | 12,071 |
| 240 | 13,894 | 12,426 | 15,361 | 12,101 | 15,686 |

TABLE 1 and corresponding FIGs. 1A-1D represent the results of the study of Example 1. Reference fluid drug levels were calculated as medians from LC-MS-MS measurements of oxycodone levels in each subject assigned to each daily dose subpopulation (column B). Raw (i.e., not normalized) medians for 80, 160, and 240 mg daily doses were 3,172; 5,245; and 8,249 ng/mL, respectively. For each of these median levels, Bonett-Price 95% confidence intervals were calculated (columns C-D). For comparison, Bonferroni-adjusted Bonett-Price 95% confidence intervals were also calculated (columns E-F). A graphical representation of these data is shown in FIGs. 1A and 1C.

Normalized median fluid drug levels were also determined as a function of urine pH, urine specific gravity, and lean body mass (column B). Normalized median urinary oxycodone levels for 80, I60, and 240 mg daily doses of controlled-release oxycodone were 5,471; 10,385; and 13,894, respectively. Bonett-Price 95% confidence intervals (columns C-D) and Bonferroni-adjusted Bonett-Price 95% confidence intervals (columns E-F) were also determined for the normalized median urinary oxycodone levels as shown in TABLE 1. A graphical representation of these data is shown in FIGs. 1B and 1D.

**Claims**

1. A method of reducing a risk of drug misuse in a subject comprising:

   determining a prescribed daily dose of a drug in the subject, wherein the drug comprises oxycodone, controlled-release oxycodone or a metabolite of oxycodone;
   measuring a concentration of the drug in urine of the subject;
   measuring parameters associated with the subject consisting of urine specific gravity, urine pH and subject lean body mass;
   calculating a normalized value (N) as a function of (i) the drug concentration and (ii) the parameters using the following equation: $[N] = [C] \times ((SG_N - 1.00)/(SG - 1.00) \times (LBW/122) \times (pH/8.50)^{0.56}$, where [C] is the raw drug concentration in ng/mL, $SG_N$ is the normalized urine specific gravity, SG is the specific gravity of the urine, LBW is the lean body weight of the subject, and pH is the pH of the urine;
   comparing the normalized value to a normalized mean or median estimate corresponding to the prescribed daily dose of the drug;
   identifying the subject at high risk of drug misuse if the normalized drug concentration falls outside the normalized mean or median estimate corresponding to the prescribed daily dose of the drug; and
   thereafter altering the daily dose of the drug prescribed to the subject.

2. The method of Claim 1 wherein the concentration of the drug is measured using LC-MS-MS or GC-MS-MS.

3. The method of Claim 1 wherein the normalized median estimate and associated upper and lower confidence levels corresponding to the prescribed daily dose of the drug are determined by:

   measuring a steady state drug concentration in urine of each of a plurality of members of a population;

measuring one or more parameters in each member;
normalizing the drug concentration for each member as a function of the one or more parameters;
determining the normalized median estimate corresponding to the prescribed daily dose of the drug from the normalized drug concentrations of the population; and
determining upper and lower confidence intervals for the normalized median estimate.


**Patentansprüche**

1. Verfahren zum Reduzieren des Risikos eines Arzneimittelmissbrauchs bei einem Subjekt, das Folgendes beinhaltet:

Bestimmen einer verordneten Tagesdosis eines Arzneimittels in dem Subjekt, wobei das Arzneimittel Oxycodon, kontrolliert freigesetztes Oxycodon oder einen Metaboliten von Oxycodon beinhaltet;
Messen einer Konzentration des Arzneimittels im Urin des Subjekts;
Messen von mit dem Subjekt assoziierten Parametern, die aus dem urinspezifischen Gewicht, Urin-pH-Wert und der mageren Körpermasse des Subjekts zusammengesetzt sind;
Berechnen eines normalisierten Werts (N) in Abhängigkeit von (i) der Arzneimittelkonzentration und (ii) den Parametern mittels der folgenden Gleichung: $[N] = [C] \times ((SGN-1{,}00)/(SG-1{,}00) \times (LBW/122) \times (pH/8{,}50)^{0{,}56}$, wobei [C] die rohe Arzneimittelkonzentration in ng/ml ist, $SG_N$ das normalisierte urinspezifische Gewicht ist, SG das spezifische Gewicht des Urins ist, LBW die magere Körpermasse des Subjekts und pH der pH-Wert des Urins ist;
Vergleichen des normalisierten Werts mit einer normalisierten mittleren oder medianen Schätzung entsprechend der verordneten Tagesdosis des Arzneimittels;
Identifizieren eines hohen Arzneimittelmissbrauchsrisikos bei dem Subjekt, wenn die normalisierte Arzneimittelkonzentration außerhalb der normalisierten mittleren oder medianen Schätzung entsprechend der verordneten Tagesdosis des Arzneimittels liegt; und
anschließend Ändern der Tagesdosis des dem Subjekt verordneten Arzneimittels.

2. Verfahren nach Anspruch 1, wobei die Konzentration des Arzneimittels mittels LC-MS-MS oder GC-MS-MS gemessen wird.

3. Verfahren nach Anspruch 1, wobei die normalisierte mediane Schätzung und dazugehörigen oberen und unteren Konfidenzniveaus entsprechend der verordneten Tagesdosis des Arzneimittels wie folgt bestimmt werden:

Messen einer Steady-state-Arzneimittelkonzentration im Urin von jedem aus einer Mehrzahl von Mitgliedern einer Population;
Messen von einem oder mehreren Parametern in jedem Mitglied;
Normalisieren der Arzneimittelkonzentration für jedes Mitglied in Abhängigkeit von den einen oder mehreren Parametern;
Bestimmen der normalisierten medianen Schätzung entsprechend der verordneten Tagesdosis des Arzneimittels anhand der normalisierten Arzneimittelkonzentrationen der Population; und
Bestimmen der oberen und unteren Konfidenzintervalle für die normalisierte mediane Schätzung.


**Revendications**

1. Procédé de réduction d'un risque de mauvaise utilisation de médicament chez un sujet, comprenant :

la détermination d'une dose quotidienne prescrite d'un médicament pour le sujet, dans lequel le médicament comprend de l'oxycodone, de l'oxycodone à libération contrôlée ou un métabolite d'oxycodone ;
la mesure d'une concentration du médicament dans l'urine d'un sujet ;
la mesure de paramètres associés au sujet consistant en la densité relative de l'urine, le pH de l'urine et la masse corporelle maigre du sujet ;
le calcul d'une valeur normalisée (N) en fonction de (i) la concentration du médicament et (ii) les paramètres, en utilisant l'équation suivante : $[N] = [C] \times ((SG_N - 1{,}00)/(SG - 1{,}00) \times (LBW/122) \times (pH/8{,}50)^{0{,}56}$, où [C] est la concentration en médicament brut en ng/mL, $SG_N$ est la densité relative de l'urine normalisée, SG est la densité relative de l'urine, LBW est la masse corporelle maigre du sujet, et pH est le pH de l'urine ;
la comparaison de la valeur normalisée à une estimée moyenne ou médiane normalisée correspondant à la dose quotidienne prescrite du médicament ;

l'identification d'un sujet présentant un risque élevé de mauvaise utilisation de médicament si la concentration en médicament normalisée se situe hors de l'estimée moyenne ou médiane normalisée correspondant à la dose quotidienne prescrite du médicament ; et

par la suite, la modification de la dose quotidienne du médicament prescrite au sujet.

2. Procédé selon la revendication 1, dans lequel la concentration du médicament est mesurée en utilisant LC-MS-MS ou GC-MS-MS.

3. Procédé selon la revendication 1, dans lequel l'estimée médiane normalisée et les niveaux de confiance supérieur et inférieur associés correspondant à la dose quotidienne prescrite du médicament sont déterminés par :

la mesure d'une concentration du médicament à l'équilibre dans l'urine de chacun d'une pluralité de membres d'une population ;

la mesure d'un ou plusieurs paramètres chez chaque membre ;

la normalisation de la concentration en médicament pour chaque membre en fonction des un ou plusieurs paramètres ;

la détermination de l'estimée médiane normalisée correspondant à la dose quotidienne prescrite du médicament à partir des concentrations en le médicament normalisé de la population ; et

la détermination d'intervalles de confiance supérieur et inférieur pour l'estimée médiane normalisée.

Fig. 1A — Raw Urinary Oxycodone

Fig. 1B — Normalized Urinary Oxycodone

Fig. 1C — Raw Urinary Oxycodone, Bonferroni-Adjusted

Fig. 1D — Normalized Urinary Oxycodone, Bonferroni-Adjusted

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Psychological Methods,* 2002, vol. 7, 370-383
- *J. Stat. Comput. Simul.,* 2001, vol. 68, 295-305
- *J. Stat. Comput. Simul.,* 2002, vol. 72, 119-124